# EUROPÄISCHE PATENTSCHRIFT

(11) **EP 0 595 124 B1**
(45) Veröffentlichungstag und Bekanntmachung des Hinweises auf die Patenterteilung: **16.04.1997**
(21) Anmeldenummer: 93116617.7
(22) Anmeldetag: 14.10.1993
(51) Int. Cl.: C07C 209/36, B01J 21/18, B01J 23/42, B01J 23/89

(54) **Verfahren zur Herstellung von chlorsubstituierten aromatischen Aminen und von Katalysatoren**
Process for the preparation of chlorosubstituted aromatic amines and of catalysts
Procédé pour la préparation d'amines chloro-substituées et de catalyseurs

(30) Priorität: 27.10.1992 DE 4236203
(43) Veröffentlichungstag der Anmeldung: 04.05.1994
(73) Patentinhaber: BAYER AG, 51368 Leverkusen (DE)
(72) Erfinder: Birkenstock, Udo, Dr., D-40882 Ratingen (DE); Kipshagen, Walter, Dr., D-51377 Leverkusen (DE); Schmidt, Herbert, D-51379 Leverkusen (DE); Schulz, Thomas-Jörg, Dr., D-51373 Leverkusen (DE); Zirngiebl, Eberhard, Dr., D-51061 Köln (DE)

(56) Entgegenhaltungen:
- EP-A- 0 002 651
- EP-A- 0 174 563
- DE-A- 3 928 329

## Beschreibung

Die vorliegende Erfindung betrifft ein verbessertes Verfahren zur Herstellung von chlorsubstituierten aromatischen Aminen durch Hydrierung von chlorsubstituierten Nitroaromaten in Gegenwart eines Edelmetall-Katalysators und die Herstellung von Edelmetallkatalysatoren.

Die Hydrierung von chlorsubstituierten aromatischen Nitroverbindungen zur Gewinnung der entsprechenden Amine in Gegenwart von Edelmetall-Verbindungen ist seit langem bekannt (siehe Ullmann, Encyclopädie der technischen Chemie, 5. Auflage, Band A2, Seite 46 (1985)). Ein wesentliches Problem bei der Durchführung dieser Reaktion ist die unerwünschte Entchlorierung, die zur Ausbeuteverringerung an chlorsubstituiertem aromatischem Amin führt. Es gibt eine Vielzahl von Verfahren, die Chlorabspaltung möglichst niedrig zu halten, z.B. durch Verwendung selektiv wirkender Edelmetall-Katalysatoren wie
- Platin und Ruthenium auf Trägermaterialien (siehe EP-OS 73 105 und US-PS 4 760 187)
und/oder die Verwendung von Zusätzen wie:
- Ammoniak oder Morpholin (siehe DE-OS 2 743 610, US-PS 3 145 231 und US-PS 3 361 819) und
- saurer Phosphorverbindungen (siehe DE-OS 2 615 079 und US-PS 4 020 107)
und/oder durch partielle Katalysatorvergiftung (siehe US-PS 4 059 627).

Weitere Möglichkeiten zur Beeinflussung der Aktivität und Selektivität von Edelmetall-Katalysatoren sind die Zugabe von Dotierungsmetallen wie:
- Silber, Nickel, Kupfer, Blei, Bismut oder Chrom (siehe US-PS 3 253 039) und
- von Chrom(III)- und Nickel(II)-Salzen (siehe US-PS 3 546 297).

Werden gleichzeitig Metalldotierungen und stickstoffhaltige Verbindungen zur Unterdrückung der Dechlorierungsreaktion eingesetzt, muß mit der erhöhten Bildung von 3,3',4,4'-Tetrachlorazobenzol (TCAB) und 3,3',4,4'-Tetrachlorazoxybenzol (TCAOB) gerechnet werden (siehe EP-OS 73 105, US-PS 4 760 187 und US-PS 3 291 032). Da TCAB und TCAOB toxisch sind, ist die Bildung dieser Produkte unerwünscht.

Die Bildung von TCAB und TCAOB kann durch die Verwendung eines Katalysators zurückgedrängt werden, der Platin und Nickel und/oder Kobalt auf einem Aktivkohle-Träger enthält (siehe DE-OS 39 28 329).

Bei den beschriebenen Verfahren werden Katalysatoren eingesetzt, die nach einer der folgenden Methoden erhalten wurden:
- Imprägnieren des Trägermaterials mit einer Edelmetallsalzlösung, gegebenenfalls mit anschließendem Verdampfen des Lösungsmittels und/oder Reduktion.
- Suspendieren des Trägermaterials in einer alkalischen Lösung und Zugabe einer Edelmetallsalzlösung, wobei Edelmetallhydroxid auf dem Trägermaterial ausfällt und gegebenenfalls anschließender Reduktion.
- Zugabe von Bicarbonat zu einer Lösung eines Edelmetallsalzes in Wasser, welches Trägermaterial suspendiert enthält, gegebenenfalls mit anschließender Reduktion.
- Aufsprühen einer Edelmetallsalzlösung auf das Trägermaterial, gegebenenfalls mit anschließender Reduktion.

Die bisher bekannten Verfahren und Katalysatoren zur Herstellung chlorsubstituierter aromatischer Amine sind noch nicht voll befriedigend hinsichtlich ihrer Selektivität und Aktivität.

Es wurde nun ein Verfahren zur Herstellung von chlorsubstituierten aromatischen Aminen durch Hydrierung von chlorsubstituierten aromatischen Nitroverbindungen in Gegenwart eines Katalysators gefunden, der Platin und Nickel und/oder Kobalt auf einem Aktivkohle-Träger enthält, das dadurch gekennzeichnet ist, daß bei der Herstellung des Katalysators das Platin auf dem Aktivkohle-Träger gleichzeitig abgeschieden und reduziert worden ist.

Gemäß dem erfindungsgemäßen Verfahren kann man beispielsweise chlorsubstituierte aromatische Nitroverbindungen der Formel (I) 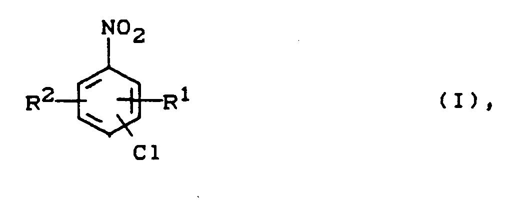 in der
- R¹ und R²: gleich oder verschieden sind und jeweils Wasserstoff, Methyl oder Chlor bedeuten,
hydrieren und so chlorsubstituierte aromatische Amine der Formel (II) erhalten 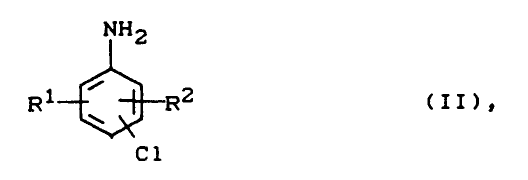 in der
- R¹ und R²: die bei Formel (I) angegebene Bedeutung haben.

Als chlorsubstituierte aromatische Nitroverbindungen können in das erfindungsgemäße Verfahren insbesondere o-, m- und/oder p-Nitrochlorbenzol, 2,4-, 2,5- und 3,4-Dichlornitrobenzol, 2,3,5- und 2,4,6-Trichlornitrobenzol und 2-Chlor-4-nitrotoluol eingesetzt und als chlorsubstituierte aromatische Amine dementsprechend insbesondere o-, m- und/oder p-Aminochlorbenzol, 2,4-, 2,5- und 3,4-Dichloranilin, 2,3,5- und 2,4,6-Trichloranilin und 2-Chlor-4-amino-toluol erhalten werden. Es können auch beliebige Gemische chlorierter aromatischer Nitroverbindungen eingesetzt und so Gemische chlorierter aromatischer Aminoverbindungen erhalten werden.

Das erfindungsgemäße Verfahren kann beispielsweise bei Temperaturen im Bereich 120 bis 160°C, vorzugsweise 130 bis 150°C und bei Drucken beispielsweise im Bereich von 10 bis 200 bar, vorzugsweise 80 bis 130 bar durchgeführt werden.

Vorzugsweise führt man das erfindungsgemäße Verfahren in Gegenwart eines Losungsmittels durch, Geeignet sind beispielsweise aliphatische Alkohole mit 1 bis 8 C-Atomen, wie Methanol, Ethanol, Propanol, Isopropanol, Butanol, Isobutanol, Pentanol, Isopentanol, Hexanol und Isohexanol, und aromatische Kohlenwasserstoffe mit 6 bis 10 C-Atomen, wie Benzol, Toluol und Xylol. Bevorzugt sind Isopropanol und Toluol.

Vorzugsweise führt man das erfindungsgemäße Verfahren in Gegenwart einer basischen Verbindung durch. Besonders bevorzugt ist hier Ammoniak, das man gasförmig oder als Lösung, z.B. als Losung in Wasser oder einem der obengenannten Losungsmittel einsetzen kann, Das Ammoniak kann im Reaktionsgemisch beispielsweise in einer Menge von 0,2 bis 5 Gew.-%, bezogen auf die eingesetzte chlorsubstituierte aromatische Nitroverbindung, vorliegen. Vorzugsweise beträgt diese Menge 0,3 bis 3 Gew.-%, besonders bevorzugt 0,7 bis 1,5 Gew.-%. Bei kontinuierlicher Fahrweise, bei der z.B. Lösungsmittel, Reaktionswasser, Katalysator und/oder Wasserstoff zurückgeführt werden, wird mit diesen Komponenten auch noch vorhandener Ammoniak zurückgeführt, der dann gegebenenfalls durch Nachdosierung auf einen gewünschten Gehalt gebracht werden kann.

Der in das erfindungsgemäße Verfahren einzusetzende Katalysator kann beispielsweise 0,3 bis 7 Gew.-%, vorzugsweise 0,5 bis 2 Gew.-%, Platin enthalten, Zusätzlich enthält er Nickel und/oder Kobalt, beispielsweise 1 bis 100 Gew.-%, vorzugsweise 10 bis 30 Gew.-% davon, bezogen auf vorhandenes Platin, Besonders bevorzugt sind Katalysatoren, die Platin und Nickel enthalten.

Das Aktivkohle-Trägermaterial für den Katalysator kann aus poröser und/oder nicht-poröser Aktivkohle bestehen.

Die Aktivkohle kann pflanzlichen oder tierischen Ursprungs und auf beliebige Weise aktiviert worden sein, beispielsweise mit Wasserdampf, Zinksulfat oder Phosphorsäure. Die Aktivkohle besteht vorzugsweise zu mindestens 80 Gew.-% aus Teilchen mit einer Korngröße von weniger als 80 µm. Derartige Aktivkohlen sind im Handel erhältlich.

Es ist ein wesentliches Merkmal der vorliegenden Erfindung, daß bei der Herstellung des Katalysators das Platin auf dem Aktivkohle-Träger gleichzeitig abgeschieden und reduziert worden ist. Man kann hier beispielsweise so verfahren, daß man den Aktivkohle-Träger zunächst in Wasser anschlämmt, dann ein wasserlösliches Reduktionsmittel für die Reduktion des Platins hinzufügt, beispielsweise Hydrazinhydrat, Ascorbinsäure, Formaldehyd oder Natriumformiat, dann alkalisch macht, beispielsweise durch Zugabe von Natronlauge oder Natriumcarbonat und schließlich langsam eine Platinsalz-Lösung, z.B. eine wäßrige H₂PtCl₆-Lösung, zutropft. Die Reihenfolge des Zusammenfügens von Aktivkohle-Träger, Wasser, wasserlöslichem Reduktionsmittel und alkalisch machendem Mittel kann auch anders sein.

Das wasserlösliche Reduktionsmittel und das alkalisch machende Mittel können jeweils z.B. als solches oder in wäßriger Lösung zugefügt werden.

Die Zutropfgeschwindigkeit der Platinsalz-Lösung wird so kontrolliert, daß das Platin sofort auf dem Aktivkohle-Träger abgeschieden und gleichzeitig zum Metall reduziert wird.

Nickel und/oder Kobalt können beispielsweise in Form einer wäßrigen Lösung ihrer Salze, z.B. der Nitrate, Chloride und/oder Sulfate, anschließend an die reduktive Abscheidung des Platins dem Gemisch zur Katalysatorherstellung zugefügt werden, Bei dieser Arbeitsweise scheiden sich Nickel und/oder Kobalt in Form ihrer Hydroxide ab.

Die reduktive Abscheidung des Platins kann beispielsweise bei Temperaturen im Bereich von 0 bis 100°C durchgeführt werden, Vorzugsweise arbeitet man bei 10 bis 30°C.

Das erfindungsgemäße Verfahren zur Herstellung chlorsubstituierter aromatischer Amine zeichnet sich durch eine hohe Selektivität aus, Es werden nur sehr wenig Nebenprodukte, z.B. TCAB, TCAOB und dehalogenierte aromatische Amine, gebildet. Der erfindungsgemäß zu verwendende Katalysator ist außerdem deutlich aktiver als bisher bekannte Katalysatoren. Das bedeutet, daß mit einer gegebenen Katalysatormenge mehr chlorsubstituierte aromatische Amine hergestellt werden können oder zur Herstellung einer bestimmten Menge chlorsubstituierter aromatischer Amine weniger Katalysator benötigt wird als bisher, Besonders stark ausgeprägt sind diese Vorteile bei der Hydrierung von 3,4-Dichlornitrobenzol zu 3,4-Dichloranilin.

Die vorliegende Erfindung betrifft weiterhin ein Verfahren zur Herstellung von Katalysatoren, die Edelmetalle auf einem Aktivkohle-Träger enthalten, das dadurch gekennzeichnet ist, daß man das Edelmetall auf dem Aktivkohle-Träger gleichzeitig abscheidet und reduziert. Als Edelmetalle kommen insbesondere Palladium und Platin in Frage. Gegebenenfalls können zusammen mit dem Edelmetall oder davor oder danach noch andere Metalle oder Metallverbindungen abgeschieden werden, z.B. Nickel, Kupfer, Blei, Wismut, Kobalt, Chrom oder Verbindungen davon.

Im einzelnen kann man das Verfahren so durchführen, wie oben für die Herstellung eines Platin und Nickel und/oder Kobalt enthaltenden Katalysators beschrieben.

Solche Edelmetallkatalysatoren können beispielsweise auch zur Reduktion von Nitronaphthalinen oder Nitrotoluolen verwendet werden.

In den folgenden Beispielen beziehen sich Angaben in Prozenten und Teilen auf das Gewicht, soweit nichts anderes angegeben ist.

### Beispiele

### I. Katalysatorherstellung

### Beispiel I-1

Zu einer Suspension von 100 Teilen Aktivkohle in 600 Teilen deionisiertem Wasser wurde eine alkalische Reduktionslösung enthaltend 6 Teile Natronlauge und 6 Teile Hydrazinhydrat in 100 Teilen Wasser zugegeben. In diese Mischung wurde eine Lösung enthaltend 2,5 Teile Hexachloroplatin(IV)-Säure (entsprechend einem Teil Platin) in 100 Teilen Wasser innerhalb von 30 Minuten eingetropft und anschließend die Mischung weitere 60 Minuten gerührt. Danach wurde eine Lösung enthaltend 0,81 Teile Nickel(II)-chlorid-hexahydrat (entsprechend 0,2 Teilen Nickel) in 100 Teilen Wasser zugegeben und die Mischung weitere 30 Minuten nachgerührt. Die Katalysatorsuspension wurde anschließend filtriert und die zurückbleibende Katalysatorpaste mit deionisiertem Wasser gewaschen bis das Waschwasser neutral reagierte. Die so hergestellte Katalysatorpaste (285 Teile mit 35 % Feststoff) enthielt 1 % Platin und 0,2 % Nickel (gerechnet als Metalle, bezogen auf trockene Kohle).

### Beispiel I-2 (Vergleichsbeispiel)

Zur Herstellung eines Katalysators nach bekannter Verfahrensweise wurde in eine Suspension von 100 Teilen Aktivkohle in 600 Teilen deionisiertem Wasser eine Lösung enthaltend 2,5 Teile Hexachloroplatin(IV)-Säure (entsprechend einem Teil Platin) in 100 Teilen Wasser innerhalb 30 Minuten zugetropft und anschließend die Mischung weitere 60 Minuten gerührt. Danach wurde eine alkalische Reduktionslösung enthaltend 6 Teile Natronlauge und 6 Teile Hydrazinhydrat in 100 Teilen Wasser zugegeben und die Mischung weitere 60 Minuten gerührt. Anschließend wurde eine Lösung enthaltend 0,81 Teile Nickel(II)-chlorid-hexahydrat (entsprechend 0,2 Teilen Nickel) in 100 Teilen Wasser zugegeben und die Mischung weitere 30 Minuten nachgerührt. Danach wurde die Katalysatorsuspension abfiltriert und die zurückbleibende Katalysatorpaste mit deionisiertem Wasser gewaschen bis das Waschwasser neutral reagierte. Die Katalysatorpaste enthielt 1 % Platin und 0,2 % Nickel (gerechnet als Metalle und bezogen auf trockene Kohle).

### Beispiel I-3

Zu einer Suspension von 100 Teilen Aktivkohle in 600 Teilen deionisiertem Wasser wurde eine alkalische Reduktionslösung enthaltend 6 Teile Natronlauge und 6 Teile Hydrazinhydrat in 100 Teilen Wasser zugegeben. In diese Mischung wurde eine Lösung enthaltend 2,5 Teile Hexachloroplatin(IV)-Säure (entsprechend einem Teil Platin) in 100 Teilen Wasser innerhalb 30 Minuten eingetropft und anschließend die Mischung weitere 60 Minuten gerührt. Die Katalysatorsuspension wurde anschließend abfiltriert und die zurückbleibende Katalysatorpaste mit deionisiertem Wasser gewaschen bis das Waschwasser neutral reagierte. Die so hergestellte Katalysatorpaste (285 Teile enthaltend 35 % Feststoff) enthielt 1 % Platin bezogen auf trockene Kohle.

### Beispiel I-4 (zum Vergleich)

Zur Herstellung eines Katalysators nach bekannter Verfahrensweise wurde in eine Suspension von 100 Teilen Aktivkohle in 600 Teilen deionisiertem Wasser eine Lösung enthaltend 2,5 Teile Hexachloroplatin(IV)-Säure (entsprechend einem Teil Platin) in 100 Teilen Wasser innerhalb 30 Minuten eingetropft und anschließend die Mischung weitere 60 Minuten gerührt. Danach wurde eine alkalische Reduktionslösung enthaltend 6 Teile Natronlauge und 6 Teile Hydrazinhydrat in 100 Teilen Wasser zugegeben und die Mischung weitere 60 Minuten gerührt. Anschließend wurde die Katalysatorsuspension abfiltriert und die zurückbleibende Katalysatorpaste mit deionisiertem Wasser gewaschen bis das Waschwassr neutral reagierte. Die Katalysatorpaste enthielt 1 % Platin bezogen auf trockene Kohle.

### II. Herstellung von chlorsubstituierten aromatischen Verbindungen

### Beispiel II-1

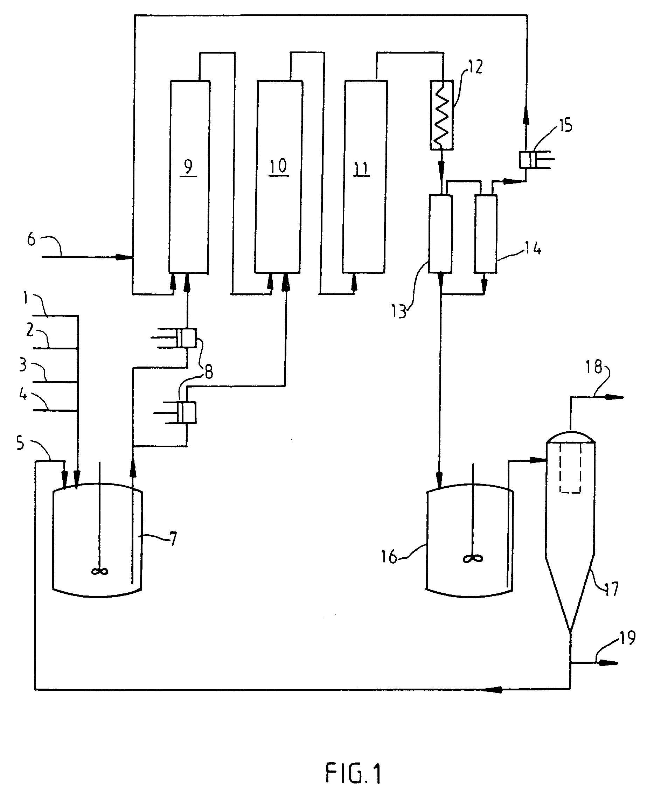

Die Bezugszeichen beziehen sich auf Fig. 1.

Die verwendete Hydrierapparatur bestand aus mehreren hintereinander geschalteten rohrförmigen Reaktoren (9), (10) und (11), die zur Abführung der Reaktionswärme mit Kühlrohren versehen waren, dem Produktkühler (12), den Abscheidern (13) und (14) und der Gasumlaufpumpe (15), mit deren Hilfe ein Wasserstoffkreislauf hergestellt und aufrechterhalten wurde.

In kontinuierlicher Arbeitsweise wurden über die Zuführungen (1), (2), (3) und (4) die zu hydrierende chlorsubstituierte aromatische Nitroverbindung (1), das Losungsmittel (2), frisch zugefügter Katalysator (3) und gegebenenfalls Ammoniak (4) in den Kessel (7) gegeben und mit in einem Reaktionsprodukt/Wasser/Lösungsmittel-Gemisch rückgeführtem Katalysator (5) vermischt.

Mittels Hochdruckpumpen (8) wurde das Gemisch anschließend in die Reaktoren (9) und (10) eingespeist und dort mit Wasserstoff (6), der in den Reaktor (9) zusammen mit rückgeführtem Wasserstoff eingebracht wurde, umgesetzt. Die Reaktionswärme wurde mit Kühlwasser abgeführte Das die Reaktoren verlassende Produkt wurde im Kühler (12) gekühlt und in den Abscheidern (13) und (14) von der Gasphase abgetrennt. Die Gasphase wurde mittels der Umlaufpumpe (15) wieder dem Reaktor (9) zugeführt. Die aus dem Hochdruckraum über die Regelventile ausgeschleuste Lösung des chlorsubstituierten aromatischen Amins wurde im Kessel (16) entgast und anschließend im Filter (17) vom Katalysator befreit. Die Lösung des chlorsubstituierten aromatischen Amins gelangte danach zur Aufarbeitung (18). Der Katalysator wurde suspendiert in einem Reaktionsprodukt/Wasser/Lösungsmittel-Gemisch wieder über Leitung (5) dem nächsten Hydrieransatz zugeführt. Gegebenenfalls kann ein Teil dieser Suspension bei (19) aus dem Kreislauf herausgenommen werden.

In die Reaktoren der vorbeschriebenen Apparatur wurden pro Stunde kontinuierlich 2 500 kg 3,4-Dichlornitrobenzol, 4 200 kg eines Isopropanol/Wasser-Gemisches, das 15 % Wasser und 0,35 % Ammoniak enthielt, 0,1 kg frischer Katalysator hergestellt gemaß Beispiel I-1 und 2 000 kg 5 % gebrauchten Katalysator enthaltende Fertigproduktlosung als Mischung eingepumpt. Im Kreislauf befanden sich somit ständig 1 bis 2 % Katalysator und 0,1 bis 0,2 % Ammoniak.

Der Wasserstoffdruck wurde auf 100 bar gehalten und die Reaktionstemperatur auf 140 bis 150°C eingeregelt. Das die Reaktoren verlassende Produkt war frei von 3,4-Dichlornitrobenzol.

Der pH-Wert der so erhaltenen Lösung von 3,4-Dichloranilin lag zwischen 9 und 12. Durch Destillation wurde das als Lösungsmittel verwendete Isopropanol/Wasser-Gemisch abgetrennt und ohne weitere Aufarbeitung wieder eingesetzt. Es enthielt Ammoniak. Verbrauchtes Ammoniak wurde durch Zugabe von gasförmigem Ammoniak in die Isopropanol-Lösung auf einen Gehalt von bis zu 0,35 Gew.-% ergänzt. Das nach weitgehender Abtrennung des Reaktionswassers in einer Scheideflasche abgetrennte rohe 3,4-Dichloranilin enthielt 99,5 % 3,4-Dichloranilin, unter 20 ppm TCAB, unter 0,2 % Monochloraniline und unter 0,1 % Anilin. Der Gehalt an TCAOB lag unter 0,1 ppm. Das Produkt hatte einen Schmelzpunkt von 71,4 bis 71,6°C.

Der Katalysatorverbrauch im Vergleich zum Verfahren gemäß der DE-OS 39 28 329 war um 60 % erniedrigt.

### Beispiel II-2 (zum Vergleich)

Es wurde wie in Beispiel II-1 beschrieben gearbeitet jedoch wurde der Katalysator durch denjenigen hergestellt nach Beispiel I-2 ersetzt.

Das nach weitgehender Abtrennung des Reaktionswassers in einer Scheideflasche abgetrennte rohe 3,4-Dichloranilin enthielt 99 % 3,4-Dichloranilin, unter 50 ppm 3,3',4,4'-Tetrachlorazobenzol, jeweils unter 0,3 Gew.-% Monochloraniline und Anilin und unter 0,1 ppm 3,3',4,4'-Tetrachlorazoxybenzol und hatte einen Schmelzpunkt von 71,5 bis 71,6°C.

Der Katalysatorverbrauch lag im Vergleich zu Beispiel II-1 um ca. 130 % höher.

### Allgemeines zu den Beispielen II-3 bis II-7

Die katalytische Aktivität von verschiedenen Katalysatoren wurde nach einer standardisierten Methode bestimmt, bei der unter Druck in einem heizbaren Rührautoklaven aus Edelstahl das Edukt jeweils in Gegenwart eines Isopropanol/Wasser-Gemisches und Ammoniak hydriert wurde.

Es wurden zwischen 4 und 8 g Katalysator zur Hydrierung von 100 g Edukt, jeweils in 300 g Isopropanol/Wasser-Gemisch (enthaltend 15 % Wasser und 10 ml wäßrigen 25 %igen Ammoniak) eingesetzt.

Der Autoklav wurde mit Wasserstoff gespült und bei Raumtemperatur auf einen Wasserstoffdruck von 100 bar eingestellt. Nach Dichtigkeitsprüfung wurden gleichzeitig der Rührer und die Heizung eingeschaltet.

Die Absorption des Wasserstoffs begann sofort unter schnellem Absinken des Wasserstoffdrucks und unter Anstieg der Reaktionstemperatur.

Nach jedem Abfallen des Wasserstoffdrucks auf 50 bar wurde erneut Wasserstoff bis auf 100 bar aufgedrückt. Die Absorption von Wasserstoff war nach einer Reaktionszeit von 5 bis 6 Minuten und einer Wasserstoffaufnahme von 160 bar beendet. Um eine vollständige Reduktion der Nitroverbindungen sicherzustellen, wurde das auf 100°C erhitzte Reaktionsgemisch noch 20 Minuten bei 100°C und einem Wasserstoffdruck von 100 bar nachgerührt.

Danach wurde unter Rühren bis auf 30°C abgekühlt und die erhaltene Lösung aromatischer Amine vom Katalysator abfiltriert (Fahrt 1).

Der abfiltrierte Katalysator wurde nochmal für weitere Hydrierungen nach oben beschriebener Arbeitsweise eingesetzt (Fahrt 2 und weitere).

Die filtrierte Aminlösung aus der ersten Fahrt und die filtrierte Aminlösung aus den folgenden Fahrten wurden mittels Gaschromatographie und/oder Hochdruckflüssigkeitschromatographie hinsichtlich der Bildung von unerwünschten Nebenprodukten untersucht.

### Beispiel II-3

Eingesetzt wurde 2,4-Chlornitrotoluol, 8 g Katalysator hergestellt gemäß Beispiel I-3 und 10 ml 25 %ige wäßrige Ammoniaklösung. Der Katalysator wurde 7 mal wiederverwendet (8 Fahrten). Nach jeder Fahrt wurde mittels Gaschromatographie die Reinheit des hergestellten 2,4-Chlortoluidins und sein Gehalt an p-Toluidin bestimmt. Die Reinheit des 2,4-Chlortoluidins lag zwischen 99,2 und 99,4 %, der Gehalt an p-Toluidin zwischen 0,32 und 0,49 %.

### Beispiel II-4 (zum Vergleich)

Es wurde verfahren wie in Beispiel II-3, jedoch wurde Katalysator hergestellt nach Beispiel I-4 eingesetzt. Die Reinheit des 2,4-Chlortoluidins lag zwischen 98,2 und 99,2 %, der Gehalt an p-Toluidin zwischen 0,47 und 1,5 %.

### Beispiel II-5

Es wurde verfahren wie in Beispiel II-4, jedoch wurde der Katalysator erhalten nach Beispiel I-1 eingesetzt. Es wurden 6 Fahrten durchgeführt. Die Reinheit des 2,4-Chlortoluidins lag zwischen 99,2 und 99,3 %, der Gehalt an p-Toluidin zwischen 0,33 und 0,36 %.

### Beispiel II-6

Eingesetzt wurden o-Nitrochlorbenzol und 4 g Katalysator hergestellt nach Beispiel I-3. Es wurden 2 Fahrten durchgeführt. Die Reinheit des erhaltenen o-Chloranilins lag zwischen 98,4 und 99 %, der Gehalt an Anilin unter 1 %.

### Beispiel II-7 (zum Vergleich)

Es wurde verfahren wie in Beispiel II-6, jedoch wurde der Katalysator hergestellt nach Beispiel I-4 eingesetzt. Die Reinheit des erhaltenen o-Chloranilins lag zwischen 96,2 und 97 %, der Gehalt an Anilin zwischen 2,9 und 3,4 %.

### Beispiel II-8

Es wurde verfahren wie in Beispiel II-3 beschrieben, jedoch wurden als Edukt 100 g 1-Nitronaphthalin und 4 g des nach Beispiel I-1 hergestellten Katalysators eingesetzt. Es wurde ohne Ammoniak-Zusatz hydriert. Der Katalysator wurde einmal zurückgeführt (insgesamt 2 Fahrten). Die Reinheit des erhaltenen 1-Naphthylamins lag zwischen 99,1 und 99,3 %.

### Beispiel II-9

Es wurde verfahren wie in Beispiel II-3 beschrieben, jedoch wurden als Edukt 80 g eines Gemisches aus o- und p-Dinitrotoluol und 4 g des nach Beispiel I-1 hergestellten Katalysators eingesetzt. Es wurde ohne Ammoniak-Zusatz hydriert. Der Katalysator wurde 3 mal zurückgeführt (insgesamt 4 Fahrten).

Die Reinheit des erhaltenen Toluylendiamins lag zwischen 99,2 und 99,25 %, der Gehalt an Hexahydro-TDA lag zwischen 0,002 und 0,005 %.

## Patentansprüche

1. Verfahren zur Herstellung von chlorsubstituierten aromatischen Aminen durch Hydrierung von chlorsubstituierten aromatischen Nitroverbindungen in Gegenwart eines Katalysators, der Platin und Nickel und/oder Kobalt auf einem Aktivkohle-Träger enthält, dadurch gekennzeichnet, daß bei der Herstellung des Katalysators das Platin auf dem Aktivkohle-Träger gleichzeitig abgeschieden und reduziert worden ist.

2. Verfahren nach Anspruch 1, dadurch gekennzeichnet, daS man chlorsubstituierte aromatische Nitroverbindungen der Formel (I) 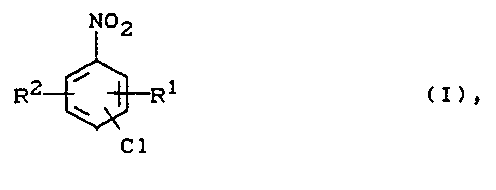 in der
R¹ und R² gleich oder verschieden sind und jeweils Wasserstoff, Methyl oder Chlor bedeuten,
hydriert und so chlorsubstituierte aromatische Amine der Formel (II) erhält 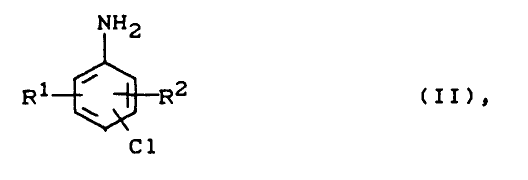 in der
R¹ und R² die bei Formel (I) angegebene Bedeutung haben.

3. Verfahren nach Ansprüchen 1 und 2, dadurch gekennzeichnet, daß der eingesetzte Katalysator 0,3 bis 7 Gew.-% Platin und, bezogen auf Platin, 1 bis 100 Gew.-% Nickel und/oder Kobalt enthält.

4. Verfahren zur Herstellung eines Katalysators der Platin auf einem Aktivkohle-Träger enthält, dadurch gekennzeichnet, daß man den Aktivkohle-Träger zunächst in Wasser anschlämmt, dann ein wasserlösliches Reduktionsmittel für die Reduktion des Platins hinzufügt, dann alkalisch macht und schließlich langsam eine Platinsalz-Lösung zutropft.

5. Verfahren nach Anspruch 4, dadurch gekennzeichnet, daß die Zutropfgeschwindigkeit der Platinsalz-Lösung so kontrolliert wird, daß das Platin sofort auf dem Aktivkohle-Träger abgeschieden und gleichzeitig zu Metall reduziert wird.

6. Verfahren nach Ansprüchen 4 und 5, dadurch gekennzeichnet, daß man Nickel und/oder Kobalt in Form einer wäßrigen Losung ihrer Salze anschließend an die reduktive Abscheidung des Platins dem Gemisch zur Katalysatorherstellung zufügt.

7. Verfahren nach Ansprüchen 4 bis 6, dadurch gekennzeichnet, daß die reduktive Abscheidung des Platins bei Temperaturen im Bereich 0 bis 100°C durchgeführt wird.

8. Verfahren zur Herstellung von Katalysatoren, die Edelmetalle auf einem Aktivkohle-Träger enthalten, dadurch gekennzeichnet, daß man das Edelmetall auf dem Aktivkohle-Träger gleichzeitig abscheidet und reduziert.

9. Verfahren nach Anspruch 8, dadurch gekennzeichnet, daß zusätzlich zum Edelmetall noch andere Metalle oder Metallverbindungen abgeschieden werden.

10. Verwendung von Edelmetallkatalysatoren hergestellt gemäß Ansprüchen 8 oder 9 für Reduktion von Nitronaphthalinen oder Nitrotoluolen.

## Claims

1. Process for the preparation of chlorine-substituted aromatic amines by hydrogenation of chlorine-substituted aromatic nitro compounds in the presence of a catalyst containing platinum and nickel and/or cobalt on an activated charcoal support, characterised in that the platinum has been simultaneously reduced and deposited on the activated charcoal support during the preparation of the catalyst.

2. Process according to Claim 1, characterised in that chlorine-substituted aromatic nitro compounds of formula (I): 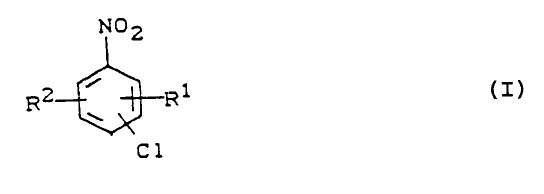 in which
R¹ and R² are identical or different and are each hydrogen, methyl or chlorine,
are hydrogenated to give chlorine-substituted aromatic amines of formula (II): 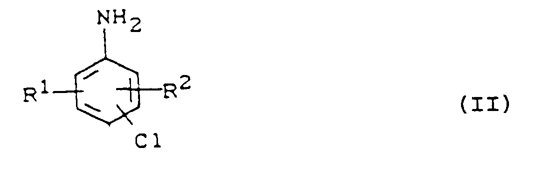 in which
R¹ and R² are as defined in formula (I).

3. Process according to Claims 1 and 2, characterised in that the catalyst used contains 0.3 to 7% by weight of platinum and 1 to 100% by weight of nickel and/or cobalt, based on platinum.

4. Process for the preparation of a catalyst containing platinum on an activated charcoal support, characterised in that the activated charcoal support is firstly suspended in water to form a slurry, a water-soluble reducing agent is then added to reduce the platinum, the mixture is then rendered alkaline and, finally, a platinum salt solution is slowly added dropwise.

5. Process according to Claim 4, characterised in that the rate of dropwise addition of the platinum salt solution is controlled so that the platinum is immediately deposited on the activated charcoal support and simultaneously reduced to the metal.

6. Process according to Claims 4 and 5, characterised in that, after the reductive deposition of the platinum, nickel and/or cobalt are added to the mixture for preparation of the catalyst, in the form of an aqueous solution of their salts.

7. Process according to Claims 4 to 6, characterised in that the reductive deposition of the platinum is carried out at temperatures in the range 0 to 100°C.

8. Process for the preparation of catalysts containing noble metals on an activated charcoal support, characterised in that the noble metal is simultaneously reduced and deposited on the activated charcoal support.

9. Process according to Claim 8, characterised in that, in addition to the noble metal, other metals or metal compounds are also deposited.

10. Use of noble metal catalysts prepared according to Claim 8 or 9 for the reduction of nitronaphthalenes or nitrotoluenes.

## Revendications

1. Procédé de préparation d'amines aromatiques chloro-substituées par hydrogénation de composés nitrés aromatiques chloro-substitués en présence d'un catalyseur qui contient du platine et du nickel et/ou du cobalt sur un support de charbon actif, caractérisé en ce que, lors de la préparation du catalyseur, le platine a été simultanément déposé et réduit sur le support de charbon actif.

2. Procédé selon la revendication 1, caractérisé en ce que l'on hydrogène des composés nitrés aromatiques chloro-substitués de formule (I) : 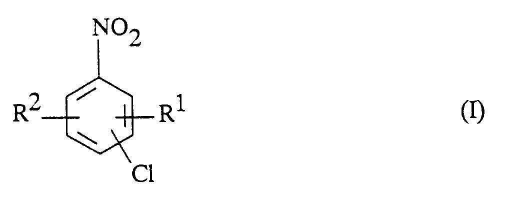 dans laquelle
R¹ et R² sont identiques ou différents et représentent chacun l'hydrogène, méthyle ou le chlore,
et l'on obtient ainsi des amines aromatiques chloro-substituées de formule (II) : 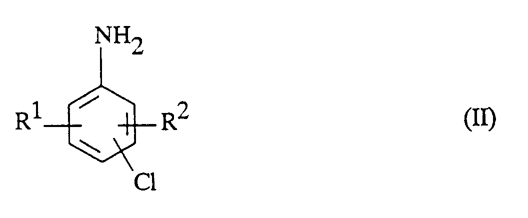 dans laquelle
R¹ et R² ont la signification indiquée au sujet de la formule (I).

3. Procédé selon les revendications 1 et 2, caractérisé en ce que le catalyseur utilisé contient 0,3 à 7 % en masse de platine et, par rapport au platine, 1 à 100 % en masse de nickel et/ou de cobalt.

4. Procédé de préparation d'un catalyseur qui contient du platine sur un support de charbon actif, caractérisé en ce que l'on met tout d'abord le support de charbon actif en suspension dans l'eau, puis on ajoute un réducteur hydrosoluble pour la réduction du platine, puis on alcalinise et enfin on ajoute lentement goutte à goutte une solution de sel de platine.

5. Procédé selon la revendication 4, caractérisé en ce que la vitesse d'addition goutte à goutte de la solution de sel de platine est commandée de telle manière que le platine se dépose immédiatement sur le support de charbon actif et est réduit simultanément en métal.

6. Procédé selon les revendications 4 et 5, caractérisé en ce que l'on ajoute au mélange pour la préparation du catalyseur du nickel et/ou du cobalt sous forme d'une solution aqueuse de leurs sels à la suite du dépôt réducteur du platine.

7. Procédé selon les revendications 4 à 6, caractérisé en ce que le dépôt réducteur du platine est réalisé à des températures situées dans le domaine de 0 à 100°C.

8. Procédé de préparation de catalyseurs qui contiennent des métaux nobles sur un support de charbon actif, caractérisé en ce que l'on dépose et réduit simultanément le métal noble sur le support de charbon actif.

9. Procédé selon la revendication 8, caractérisé en ce que, en plus du métal noble, d'autres métaux ou composés métalliques sont déposés encore.

10. Utilisation de catalyseurs à base de métaux nobles préparés selon la revendication 8 ou 9 pour la réduction de nitronaphtalènes ou de nitrotoluènes.
